# EUROPEAN PATENT APPLICATION

(11) **EP 2 332 596 A1**
(43) Date of publication of application: **15.06.2011**
(21) Application number: 10190296.3
(22) Date of filing: 08.11.2010
(51) Int. Cl.: A61M 5/142, A61M 5/168

(54) **Infusion pump module**

(30) Priority: 12.11.2009 JP 2009259143
(71) Applicant: Ricoh Company Ltd., Tokyo 143-8555 (JP)
(72) Inventor: Tomoyama, Takashi, Tokyo 143-8555 (JP); Kamada, Teruki, Tokyo 143-8555 (JP); Sugimoto, Yasunori, Tokyo 143-8555 (JP)
(74) Representative: Schwabe - Sandmair - Marx

(57) **Abstract**

An infusion pump module for transferring fluid includes a fluid inlet part having an inlet to receive the fluid, a fluid outlet part having an outlet to discharge the fluid, a pump unit having a piezoelectric element, and a flow rate sensor to measure a flow rate of the fluid flowing inside the module, wherein the fluid inlet part, the fluid outlet part, the pump unit, and the flow rate sensor are connected together to cause the fluid received by the fluid inlet part to be discharged from the fluid outlet part, and the fluid inlet part, the fluid outlet part, the pump unit, and the flow rate sensor connected together form a single continuous flow channel.

## Description

### BACKGROUND OF THE INVENTION

### 1. Field of the Invention

The disclosures herein generally relate to an infusion pump module provided with a flow rate sensor to measure the flow rate of fluid being transferred.

### 2. Description of the Related Art

In medical practice, for example, a medical solution for infusion into a patient may need to be transferred. To this end, a fluid transfer pump module is used that includes a piezoelectric-element-based pump and an oscillator circuit for driving the piezoelectric element (see Japanese Patent Application Publication No. 8-303352, for example). In such a fluid transfer pump, a voltage signal having predetermined frequency is applied to vibrate the piezoelectric element, thereby pushing a medical solution in a flow channel for fluid transfer.

Further, a flow rate sensor is attached to a fluid conduit such as a tube connected to the fluid transfer pump, and the oscillator circuit is controlled based on the flow rate measured by the flow rate sensor. This allows the amount of fluid being transferred to be controlled.

In the above-noted structure, however, the fluid transfer pump, the fluid conduit, and the flow rate sensor attached to the fluid conduit need to be provided separately. The complete system is then constructed by connecting these parts. The size of the system thus becomes voluminous, thereby restricting the choice of place at which the system is installed.

Accordingly, there may be a need for an infusion pump module that can control the flow rate of fluid being transferred and that is compact so as not to restrict the choice of place of installment.

### SUMMARY OF THE INVENTION

It is a general object of at least one embodiment of the present invention to provide an infusion pump module that substantially eliminates one or more problems caused by the limitations and disadvantages of the related art.

In one embodiment, an infusion pump module for transferring fluid includes a fluid inlet part having an inlet to receive the fluid, a fluid outlet part having an outlet to discharge the fluid, a pump unit having a piezoelectric element, and a flow rate sensor to measure a flow rate of the fluid flowing inside the module, wherein the fluid inlet part, the fluid outlet part, the pump unit, and the flow rate sensor are connected together to cause the fluid received by the fluid inlet part to be discharged from the fluid outlet part, and the fluid inlet part, the fluid outlet part, the pump unit, and the flow rate sensor connected together form a single continuous flow channel.

According to at least one embodiment, the infusion pump module can control the flow rate of fluid being transferred, and can be made compact so as not to restrict the choice of place of installment.

### BRIEF DESCRIPTION OF THE DRAWINGS

Other objects and further features of embodiments will be apparent from the following detailed description when read in conjunction with the accompanying drawings, in which:
Fig. 1 is a drawing illustrating a main part of an infusion pump module according to an embodiment;
Fig. 2 is a drawing illustrating the entire configuration of the infusion pump module according to the embodiment;
Fig. 3 is a drawing illustrating an example of the configuration of a system utilizing the infusion pump module according to the embodiment;
Fig. 4 is a drawing illustrating the arrangement of a body, a pump, and a flow rate sensor of the infusion pump module according to the embodiment;
Fig. 5 is a drawing illustrating the configuration of an assembled infusion pump module, a direction of assembling, and a direction in which electric power is supplied according to the embodiment;
Fig. 6 is a drawing illustrating connection points between the pump, the flow rate sensor, and the body in the infusion pump module according to the embodiment;
Fig. 7 is a drawing illustrating an example of the configuration of a pump used in the infusion pump module according to the embodiment; and
Fig. 8 is a drawing illustrating the main structural components of the infusion pump module for the purpose of explaining materials of the components.

### DESCRIPTION OF THE PREFERRED EMBODIMENTS

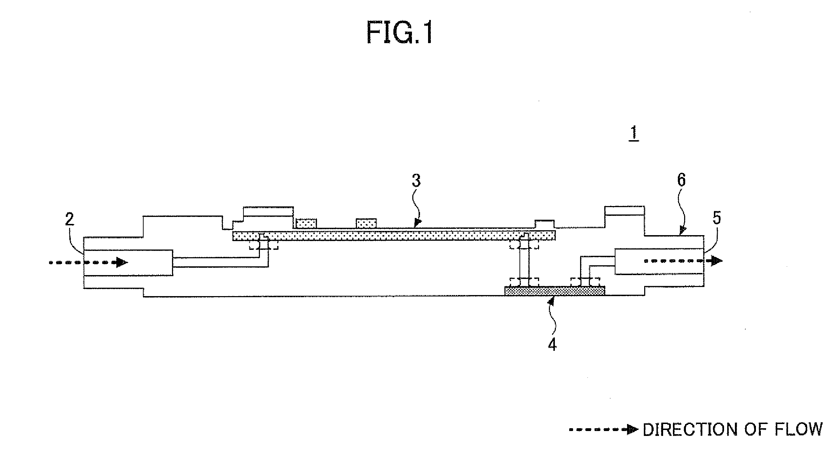

In the following, embodiments for carrying out the invention will be described by referring to the accompanying drawings. In the following, a description will be given of the configuration of a flow channel of an infusion pump module according to an embodiment. Fig. 1 is a drawing illustrating the configuration of a main part 1 of the infusion pump module. As illustrated in Fig. 1, the main part 1 of the infusion pump module includes a fluid inlet 2, a pump 3, a flow rate sensor 4, a fluid outlet 5, and a body 6. In the infusion pump module, fluid inflowing through the fluid inlet 2 is transferred through the pump 3 to the flow rate sensor 4 for outflow from the fluid outlet 5. The pump 3 includes a piezoelectric element. A voltage having predetermined frequency is applied to the piezoelectric element to cause vibration, thereby exerting the pump function.

As illustrated in Fig. 1, the pump 3 and the flow rate sensor 4 are placed to face each other, with a structure of the body (molded component) 6 placed therebetween, to constitute a flow channel of the infusion pump module by use of a single integral structure. Namely, the component that serves as the body 6 to form a flow channel is implemented as a single component. With the use of this configuration, the entire length of the infusion pump module is made short. In Fig. 1, the pump 3 and the flow rate sensor 4 are arranged in the order named from upstream to downstream. Alternatively, the flow rate sensor 4 and the pump 3 may be arranged in the order named from upstream to downstream.

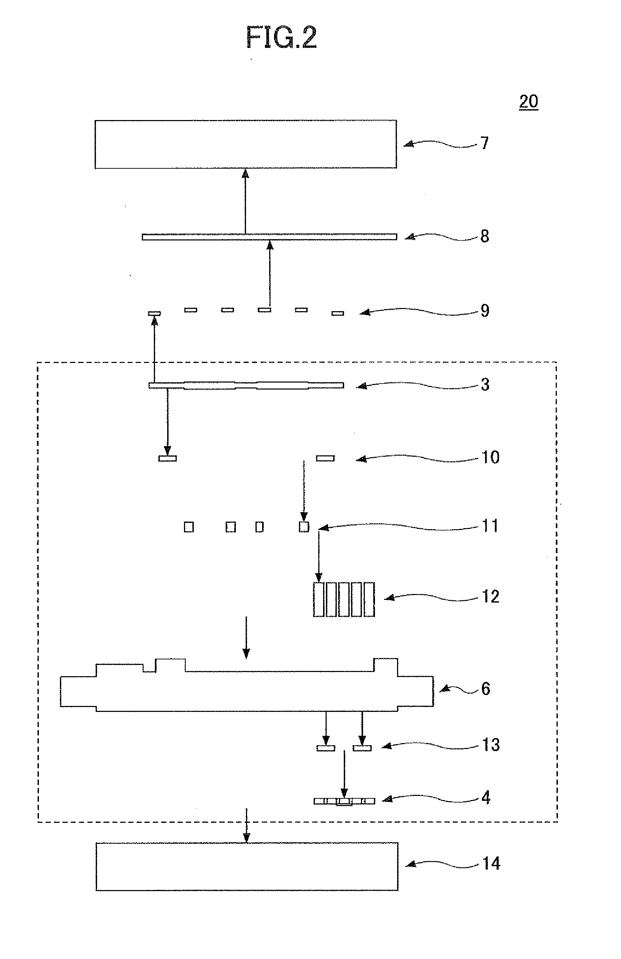

In the following, an entire configuration of the infusion pump module will be described with reference to Fig. 2. Fig. 2 is a drawing illustrating the components of an infusion pump module 20. As illustrated in Fig. 2, the infusion pump module 20 includes covers 7 and 14, a PCB (Printed Circuit Board) 8, contact members (gasket) 9, the pump (inclusive of the piezoelectric element) 3, seal members (O-rings) 10 and 13, a probe 11, a probe 12, the body 6, and the flow rate sensor 4.

The cover 7 and the cover 14 are connected to each other in a tightening manner to form a module, thereby preventing the PCB 8 from dropping off and also protecting other components. The PCB 8 transmits drive power from an external controller to the pump 3 and the flow rate sensor 4. The contact members 9 serve to transmit electric power from the PCB 8 to the piezoelectric element of the pump 3. The pump 3 includes a fluid chamber for storing fluid being transferred and the piezoelectric element serving as a power source for transferring fluid inside the fluid chamber. The pump 3 drives the piezoelectric element by the electric power supplied from the PCB 8 through the contact members 9, thereby transferring the fluid.

The seal members 10 and 13 serve to prevent the leakage of transferred fluid and the intrusion of a foreign substance. The seal members 10 and 13 are inserted into 0-ring grooves formed in the body 6, and the pump 3 and the flow rate sensor 4 are placed thereon, respectively. The seal members 10 and 13 are squashed and deformed to a proper degree by being placed between the body 6 and the pump 3 or between the body 6 and the flow rate sensor 4, thereby tightly connecting between the flow channel of the body 6 with the flow channels of the pump 3 and flow rate sensor 4 to prevent the leakage of fluid at the connection points. The seal members 10 and 13 may preferably be 0-rings.

The probe 11 is connected to electrodes provided on the upper and lower surfaces of the pump 3 for supplying electric power. The probe 12 serves to electrically connect between the PCB 8 and electrodes for driving and controlling the flow rate sensor 4. The body 6 is a component for forming a flow channel that transfers fluid. The body 6 has the inlet (i.e., fluid inlet 2) of the flow channel and the outlet (i.e., fluid outlet 5) of the flow channel in the infusion pump module 20. The body 6, the pump 3, and the flow rate sensor 4 are connected together, thereby forming a single continuous flow channel from the inlet to the outlet. In other words, the body 6 that is a unitary seamless component forms a single continuous flow channel from the fluid inlet to the fluid outlet, except where the pump 3 and the flow rate sensor 4 are provided. The body 6 has the O-ring grooves for receiving the seal members 10 and 13 in the surfaces that meet the pump 3 and the flow rate sensor 4, respectively. The body 6 also has grooves for holding the probe 11 and the probe 12.

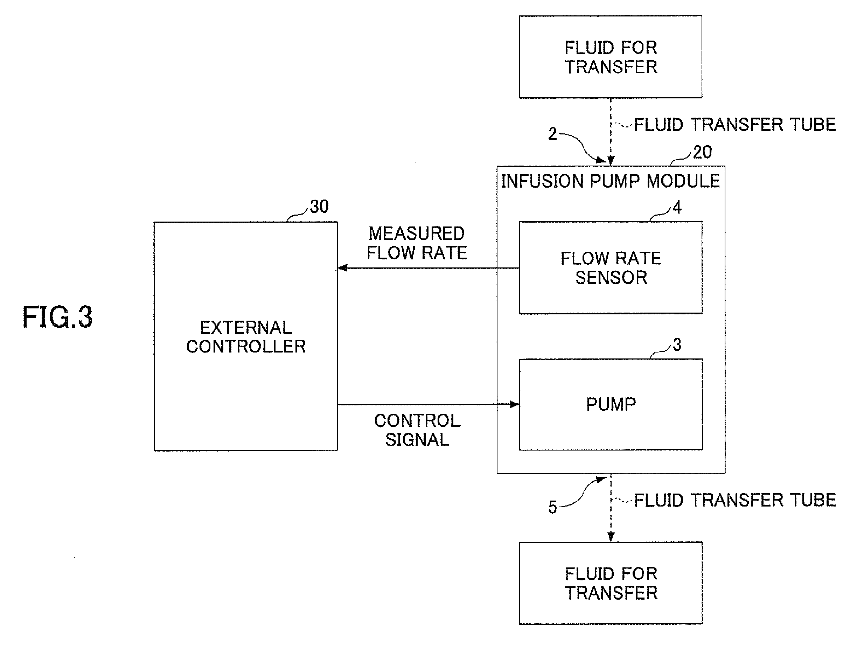

The flow rate sensor 4 measures the flow rate of fluid being transferred by the pump 3. The flow rate sensor 4 transmits data indicative of the measured flow rate to an external controller 30 (see Fig. 3) through the PCB 8. In response to the data indicative of the measured flow rate supplied from the flow rate sensor 4, the external controller 30 transmits a control signal to the piezoelectric element of the pump 3. The cover 14 and the cover 7 are connected to each other in a tightening manner to form a module, thereby preventing the flow rate sensor 4 from dropping off and also protecting other components.

In the infusion pump module 20 constructed by assembling the above-noted components, the pump 3 exerts the function to transfer fluid by vibrating the piezoelectric element, and the body 6 serves as the flow channel through which the fluid is transferred.

In the following, an example of usage of the infusion pump module 20 will be described with reference to Fig. 3. Fig. 3 is a drawing illustrating an example of the configuration of a system that includes the infusion pump module 20 and the external controller 30. As illustrated in Fig. 3, the infusion pump module 20 transfers fluid through fluid transfer tubes connected to the fluid inlet 2 and to the fluid outlet 5, respectively. In this system, the infusion pump module 20 is connected to the external controller 30, so that the data indicative of a flow rate of fluid measured by the flow rate sensor 4 is reported to the external controller 30.

The external controller 30 generates a control signal based on the received data indicative of the measured flow rate, and sends the control signal to the pump 3 (i.e., to the piezoelectric element). The piezoelectric element of the pump 3 vibrates in response to the control signal, so that the pump 3 exerts the pump function to transfer the fluid. In the external controller 30, the relationship between the received data indicative of measured flow rate and the control signal generated in response thereto may properly be adjusted.

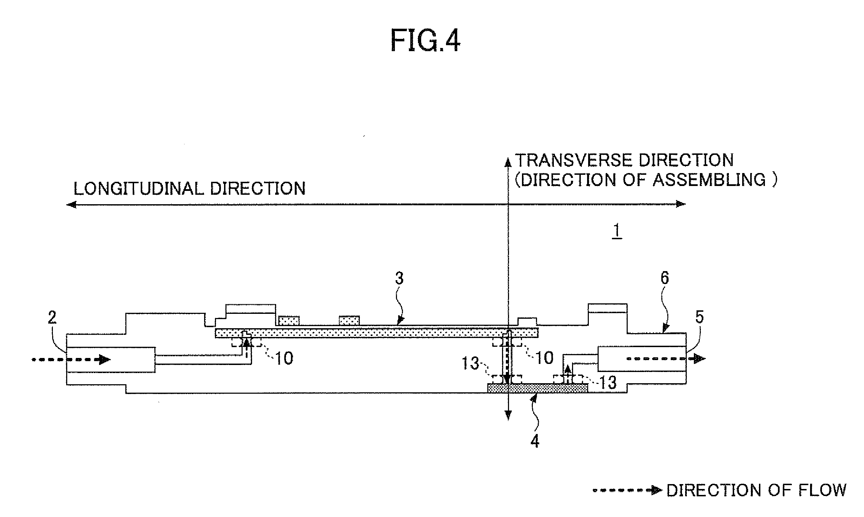

In the following, the details of the configuration of the infusion pump module 20 will be described with reference to Fig. 4. Fig. 4 is a drawing illustrating the configuration of the assemblage of the body 6, the pump 3, and the flow rate sensor. As illustrated in Fig. 4, the pump 3 and the flow rate sensor 4 are fixedly mounted to the body 6, with the O-rings (i.e., seal members 10 and 13) being used as waterproof seal members to secure the flow channel. O-rings are commercially available products, and may be acquired at low cost compared to resin sheets.

The fluid inlet and outlet of the pump 3 and the fluid inlet and outlet of the flow rate sensor 4 are situated at opposite surfaces of the body 6 that forms a flow channel, and, more specifically, the fluid outlet of the pump 3 and the fluid inlet of the flow rate sensor 4 are situated on the same axis extending in the direction in which the components are assembled, thereby making the size of the infusion pump module 20 compact. In particular, the length of the infusion pump module in its longitudinal direction is made short.

Moreover, the pump 3 and the flow rate sensor 4 are assembled by compressing the seal members 10 and 13 for the purpose of preventing fluid leakage. In general, the manufacturing variations of individual components are added together, which may result in a gap appearing between the assembled components, or may result in a distortion occurring in a case component or the like, thereby creating a risk of causing fluid leakage. In the infusion pump module 20, however, 0-rings may be used as the seal members 10 and 13, thereby eliminating the need to control pressure at the time of compression for assembling. There is also no need to use an inspection device during the assembly process. This arrangement thus reduces the assembly cost.

High process precision may be required for the fluid inlet and the fluid outlet, and may also be required for the pump, the flow rate sensor, and the body serving as a casing. Process precision for other components may be set lower. This allows process precision for individual components to be optimized, thereby reducing the cost of molds, shortening molding cycles, and reducing the cost of molded products.

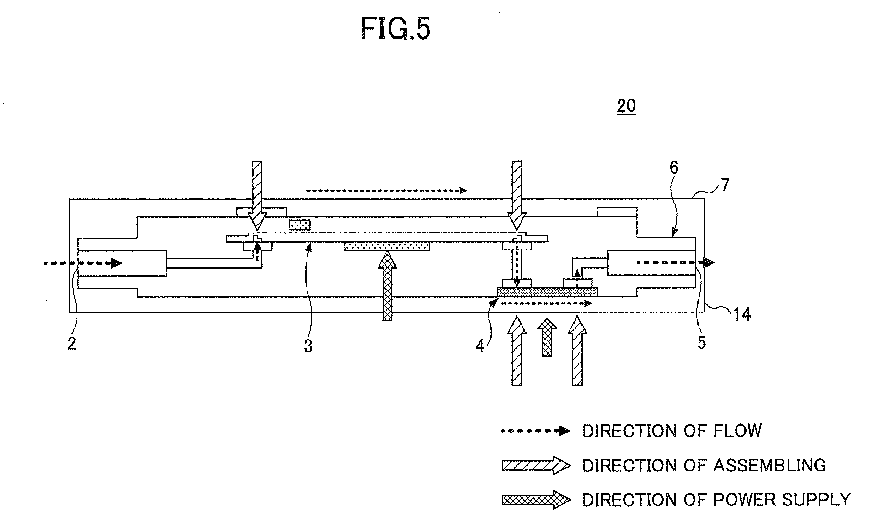

Fig. 5 is a drawing illustrating the configuration of assembled components, a direction in which the components are assembled and subjected to pressure, and a direction in which electric power is supplied. As illustrated in Fig. 5, the main components of the infusion pump module 20 are pressed, without the need to control pressure. In the assembling process, components are assembled upon components in one direction (e.g., from an upper side to a lower side), thereby improving the ease of assembling the components. This serves to simplify the assembly facility. Further, with the covers 7 and 14 connected together in a tightening manner, the individual components of the infusion pump module 20 are kept in the connected state at the respective connection points by the force applied by the covers along the axis extending in the direction of assembling (i.e., in the transverse direction of the infusion pump module 20).

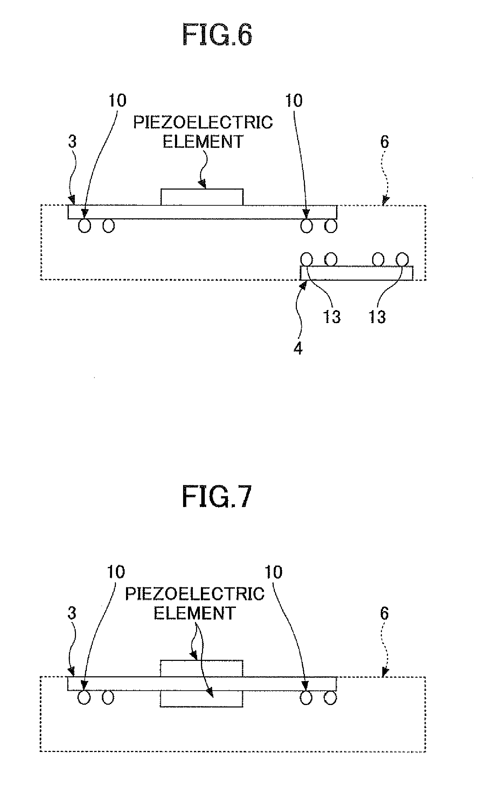

In the following, a description will be given of an advantage of the configuration of the infusion pump module 20 by referring to Fig. 6 and Fig. 7. Fig. 6 is a drawing illustrating the connection points between the body 6 and either one of the pump 3 and the flow rate sensor 4. Fig. 7 is a drawing illustrating an example of the configuration of the pump 3 used in the infusion pump module 20. As illustrated in Fig. 6, with O-rings used as the seal members 10 and 13, the pump 3 and the flow rate sensor 4 are pressed against the rigid body 6, so that there is no need to control pressure in the infusion pump module 20. Further, space is secured around the pump 3 by use of the O-rings as the seal members 10 and 13, which makes it possible to easily change the layout of one or more piezoelectric elements of the pump 3 in the infusion pump module 20. As illustrated in Fig. 7, the piezoelectric-element layout of the pump 3 may be such that piezoelectric elements are situated on both the upper side and the lower side of the pump 3. Such a layout is possible because the shape of the body 6 can be changed to accommodate one or more piezoelectric components. Further, an alternative piezoelectric-element layout of the pump 3 may be such that a piezoelectric element is situated only on the lower side of the pump 3.

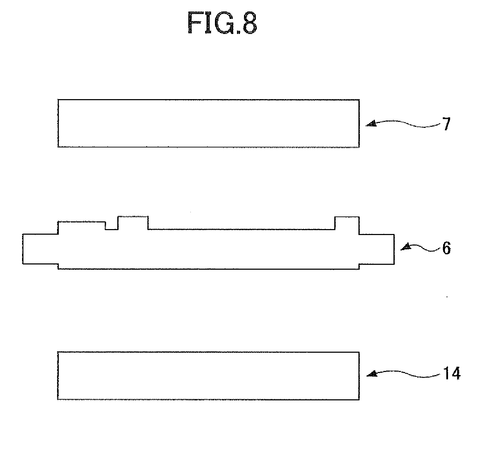

In the following, the materials of the components constituting the infusion pump module 20 will be described by referring to Fig. 8. Fig. 8 is a drawing illustrating the main structural components of the infusion pump module 20 for the purpose of explaining materials of the components. In the infusion pump module 20 that is used in medical practice, the material of the components that form a flow channel, such as the main part 1 or the body 6, may be a medical grade material or a material that is inactive to fluid transferred by the infusion pump module 20. Other components that do not form a flow channel, such as the covers 7 and 14, do not come in contact with fluid transferred by the infusion pump module 20. Accordingly, no material restriction may be imposed on these components. For example, a resin material available at low cost may be used for the covers, thereby reducing the cost of components.

Further, the present invention is not limited to these embodiments, but various variations and modifications may be made without departing from the scope of the present invention.

## Claims

1. An infusion pump module for transferring fluid, **characterized by** comprising:
a fluid inlet part having an inlet to receive the fluid;
a fluid outlet part having an outlet to discharge the fluid;
a pump unit having a piezoelectric element; and
a flow rate sensor to measure a flow rate of the fluid flowing inside the module,
wherein the fluid inlet part, the fluid outlet part, the pump unit, and the flow rate sensor are connected together to cause the fluid received by the fluid inlet part to be discharged from the fluid outlet part,
and the fluid inlet part, the fluid outlet part, the pump unit, and the flow rate sensor connected together form a single continuous flow channel.

2. The infusion pump module as claimed in claim 1, wherein the pump unit and the flow rate sensor are arranged to at least partially overlap each other in a longitudinal direction of the module.

3. The infusion pump module as claimed in claim 2, wherein the pump unit and the flow rate sensor are arranged in parallel in the longitudinal direction of the module.

4. The infusion pump module as claimed in claim 3, further comprising
a connecting flow channel part that connects between the pump unit and the flow rate sensor,
the fluid inlet part, the fluid outlet part, the pump unit, the flow rate sensor, and the connecting flow channel part are kept in a connected state by a force applied in a transverse direction of the module.

5. The infusion pump module as claimed in claim 4, wherein a point at which the pump unit and the connecting flow channel part are connected to each other and a point at which the flow rate sensor and the connecting flow channel part are connected to each other are positioned on a same axis extending in the transverse direction.

6. The infusion pump module as claimed in claim 5, wherein O-rings are used at connection points between the fluid inlet part, the fluid outlet part, the pump unit, the flow rate sensor, and the connecting flow channel part.

7. The infusion pump module as claimed in claim 6, wherein one or more materials that form the flow channel are made of one or more medical grade materials or one or more materials inactive to the fluid.

8. The infusion pump module as claimed in claim 1, wherein the fluid inlet part and the fluid outlet part are part of a unitary seamless component that has the single continuous flow channel extending from the fluid inlet part to the fluid outlet part, except where the pump unit and the flow rate sensor are provided along the single continuous flow.
